# EUROPEAN PATENT APPLICATION

(11) **EP 1 570 879 A2**
(43) Date of publication of application: **07.09.2005**
(21) Application number: 05004447.8
(22) Date of filing: 01.03.2005
(51) Int. Cl.: A61N 1/30

(54) **Improved buffer gel for iontophoresis electrodes**

(30) Priority: 03.03.2004 US 708432
(71) Applicant: IOMED, INC., Utah 84120 (US)
(72) Inventor: Plummer, Thomas, Salt Lake City Utah 84121 (US); Szlek, Malgorzata, Salt Lake City Utah 84106 (US); Beck, Jon E., Salt lake City Utah 84109 (US)
(74) Representative: Ostertag, Ulrich

(57) **Abstract**

An iontophoretic delivery device utilizing a polymeric gel matrix to ameliorate pH deviation within the device wherein the pH of the polymeric gel matrix is buffered at select pH ranges in order to reduce skin irritation. The polymeric gel matrix is housed within the iontophoretic delivery device as a buffering agent wherein the matrix provides a suitable physiological pH level above 4.0 and particularly the pH level deviates between approximately 4.1 to approximately 4.9, and preferably at the pH 4.5 for an effective operation of the iontophoretic delivery of a medicament to treat affected areas of a living subject's body.

## Description

### BACKGROUND OF THE INVENTION

### 1. Field of the Invention

The present invention relates in general to iontophoretic delivery devices, compositions, and methods thereof. More particularly, this invention relates to an improvement in a pH buffered gel for use in iontophoretic process, wherein the gel is composed in such a way that the pH is maintained within a narrow physiologically safe range during iontophoretic procedures.

### 2. Background Art

It is widely known by those skilled in the art that the process of iontophoresis can be used in applying medication locally through a patient's skin as well as for use in delivering medicaments to the eyes and ears. This technique involves the application of active delivery of medications in which transport of ionized compounds into tissues (i.e. skin, mucous membrane, or nails) is enhanced by an externally applied electric field. Accordingly, the process of transdermal iontophoretic delivery works in such a way that various substances can be administered to a patient without the necessity of a hypodermic injection and without the associated problems, such as pain, the risk of infection, and trauma to the patient - just to name a few.

The application of iontophoretic delivery has been tested for use in treatments such as the application of local anesthetics, the application of medication for treatment of skin disorders, and the application of other limited types of medications in order to achieve a localized effect. As mentioned above, the iontophoretic technique involves externally applying an electric field to the active delivery of medicaments; in essence, this technique drives ionized compounds through the skin. Thus, positively charged ions are driven into the skin at the anode of an electrical system, and negatively charged ions are driven into the skin at the cathode of the electrical system. For example, positively charged ions such as zinc, copper, alkaloids, certain anesthetics, and certain vasodilating drugs are introduced into the skin or the mucous membranes from the anodic side. On the other hand, a negatively charged drug, such as salicylate, fluoride, penicillin, and insulin can be driven into the skin using the cathodic side.

Iontophoretic delivery of medicaments can provide significant benefits over other methods of delivery, such as electro-osmosis, oral intake, and injections - just to name a few. Advantages of applying the iontophoretic delivery of medicaments include, among others, rapid results, non-invasive drug entry into the skin and underlying tissues, avoidance of pain associated with tissue distortion during injections, elimination of the risk of infection, and controllable rate of drug administration. One example of drugs, which exhibits localized effects upon iontophoresis through the skin, is local anesthetics. The iontophoretic technique allows the local anesthetics to effect localized areas in the skin of the patients rapidly, and this technique avoids the issue of pain associated with injections and eliminates the risk of infection. Other example includes a medicament delivered iontophoretically into the circulatory system. The medicament delivered iontophoretically can be quickly absorbed into the circulatory system; therefore, the process of iontophoretic delivery is capable of delivering an effective amount of medicaments necessary for the patients.

When a medicament is taken orally, though effective in some level, the result is uncertain because in order for the medicament to be effective when taken orally, the medicament must be completely absorbed through the digestive tract. Since the oral intake of the medicament through the digestive tract varies greatly from individual to individual, this causes uncertainty in the amount of an orally ingested drug needed to achieve the desired therapeutic effect. Accordingly, the application of iontophoretic techniques effectively eliminates the uncertainty of whether orally taken drugs have fully absorbed through the digestive tract because this technique allows for a rapid result by transdermally delivering the medicament to an intended localized area of a patient. In addition, the iontophoretic delivery application allows for an administration of medicaments over a sustained period of time without invasion of the body. To achieve this through other methods of delivery, such as injections, requires someone to periodically administer the medicaments, which can be cumbersome to say the least because this requires constant monitoring of the patient's system and the necessity to ensure that the patient is receiving a certain constant level of medicament within the patient's system. Therefore, the iontophoretic delivery is non-invasive to a patient, and this delivery ensures that pain and infections, associated with injecting the medicament to a patient's skin, are ameliorated in the process. Despite these potential benefits for iontophoretic delivery applications, limitations in the presently available iontophoretic delivery systems do not make a sustained iontophoretic technique practical to use.

One potential problem with the iontophoretic techniques is skin irritation. It has been known that applying electric current through skin under certain conditions may cause skin irritation. Additionally, other factors can cause, or at least contribute to, skin irritation during the iontophoretic delivery. Under certain conditions, water hydrolysis tends to occur at the interface between the electrode and the drug solution or electrolyte salt solution. The products of water hydrolysis (i.e., hydronium ions are produced by water hydrolysis at the anode and hydroxyl ions are produced by water hydrolysis at the cathode) compete with the drug ions of like charge for delivery into the skin, thereby altering skin pH. Since highly basic or acidic solutions in contact with the skin surface are known to damage tissue, the pH altering effects of the iontophoretic delivery device can cause skin irritation.

In order to ameliorate this problem of pH deviation, which causes skin irritation, iontophoretic applications have used silver/silver chloride or ion exchange resins to control the pH of the drug matrix during the process of iontophoresis delivery. Although use of silver/silver chloride ameliorates the hydrolysis problem, problems exist relative to costs associated with the metal/metal chloride and associated manufacturer costs.

Ion exchange resins have also been used to neutralize the hydronium and/or hydroxyl ions. This, in turn, can mitigate skin irritation associated with the hydrolysis problem. However, ion exchange resins require processing insoluble resin powders, and it is without certainty that the resins will be homogeneous with regards to pH control throughout the entire iontophoretic delivery system. Therefore, lack of homogeneity in the delivery system can cause pH deviation within the delivery system, and this can lead to skin irritation as well.

It is thus an object of this invention to construct a homogeneous rehydratable gel matrix to provide a suitable alternative for existing metal/metal chloride and ion exchange resin systems.

It is further an object of this invention to construct a homogeneous rehydratable gel matrix to resist extreme changes in pH, which in turn will prevent skin irritation associated with operation of iontophoretic delivery device.

### SUMMARY OF THE INVENTION

The present invention disclosed herein is an iontophoretic delivery device, comprising a polymeric gel matrix having a medicament, a buffering agent, a viscosity enhancer, a rehydrating agent, and an active electrode associated therein to treat affected areas of a living subject without any negative harms such as skin irritation and/or skin burns associated with the prior art iontophoretic delivery systems. The buffering agent associated with the polymeric gel matrix controls a pH of the delivery system to maintain the pH above 4.0. In particular, the matrix adjusts the pH of the delivery system between approximately 4.1 and approximately 4.9, and preferably, at pH 4.5, for effectively delivering the medicament to treat affected areas of a living body's subject.

The present invention is also directed to a method for treating inflammation, immune suppression, etcetera, which comprises the steps of: (a) associating a medicament with a matrix in an iontophoretic delivery device; (b) providing an effective amount of pH buffering agents to the matrix; (c) adding a viscosity enhancer to attain high buffer capacity in the matrix; (d) adding a rehydrating agent to the matrix to facilitate homogeneous hydration within the matrix; (e) positioning at least a portion of the iontophoretic device on an affected area of a living subject; and (f) iontophoretically delivering the medicament to the affected area of the living subject while minimizing skin inflammation and/or pain.

In a preferred embodiment of the present invention, a polymeric gel matrix is comprised of poly (methylvinyl ether maleic acid) and sufficient glycine to adjust the pH to between approximately 4.1 and approximately 4.9; typically at 4.4-4.5. Exemplary materials such as aforementioned are available under the trade name Gantrez® (ISP Technologies, Inc.). The sufficient glycine to be used will be discussed more in detail below, and it will be familiar to those having ordinary skill in the art having this disclosure before them as a guide.

The polymer as used in this preferred embodiment is represented by the following chemical structures: 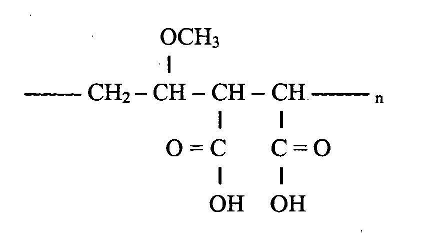 (picture slightly distorted to maintain pictorial clarity). As would be readily understood to those having ordinary skill in the art, the water-soluble or water-dispersible polymers useful in the practice of the present invention may comprise any natural or synthetic polymer within that broad class.

Amino acids used to neutralize the polymer can be any one of soluble amino acids, and the amino acid as used in this preferred embodiment is represented by the following chemical structures: 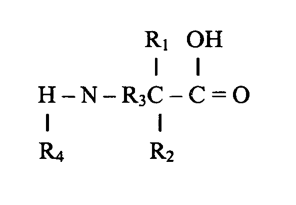 (picture slightly distorted to maintain pictorial clarity), wherein R1, R2, and R4 are the same or different and comprise methyl, ethyl, propyl, butyl, aromatic and combinations thereof; and R3 is the same or different and comprises a direct carbon-nitrogen bond, methylene, propylene, butylene, aromatic and combinations thereof. Amino acids used to neutralize the polymer can be any one of soluble amino acids. Viscosity of the matrix can be adjusted with a number of viscosity enhancers including but not limited to hydroxy ethyl cellulose known as NATROSOL® (Hercules) and hydroxy propyl cellulose, known as KLUCEL® (Astro Chemicals Inc.). Additional components are added to facilitate hydration of the dried resin. These components include but are not limited to Waterlock A220 and Tween-20 as wetting agents.

In the above embodiment, the step of providing the buffering agents includes the step of providing Gantrez® S-97 and Glycine wherein these work in concert to assure minimal deviations from pH of approximately 4.5. Gantrez® S-97 can be used in solution with concentrations of 0.1% to 30% by weight, preferably 2% to 5%, and most preferably 3.0%. The glycine may be used in concentration of 0.1% to 50%, preferably 6.0% to 9.0%, and most preferably 6.5%. The Natrosol® 250 may be used in solution at concentrations ranging from 0.01% to 4.0%, preferably 0.15% to 0.3%, and most preferably 0.25%. The wetting agents, Waterlock A220 and Tween-20, can be used in concentrations of 0.01% to 10.0%, preferably 0.25% to 0.5%, but most preferably 0.5% for the Waterlock A220 and 0.25% for the Tween-20.

In a preferred embodiment of the present invention, the step of providing a medicament is selected from the group consisting of the following chemical structures:

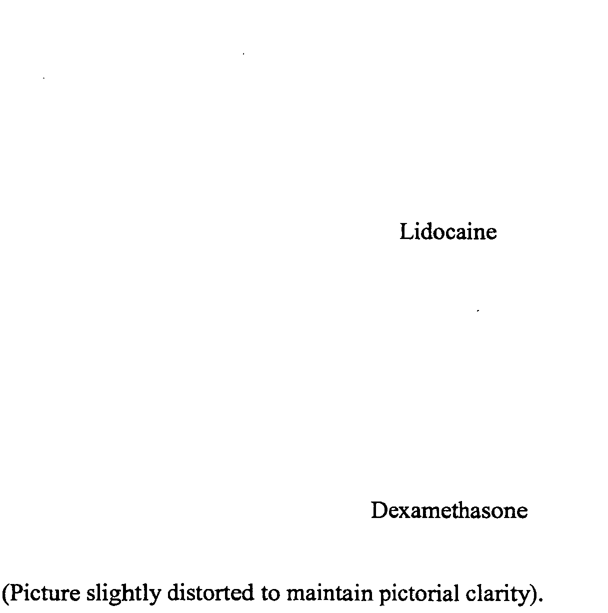

In yet another preferred embodiment, acrylate polymers such as the Carbopol® series of polymers may be substituted for the Gantrez® S-97. These acrylate polymers respond to soluble amino acid neutralization in a manner similar to Gantrez®. For instance, a 1.0% solution of Carbopol® when mixed with 1.0% glycine renders a gel having a viscosity of 16,750 cps and a pH of 4.1. Likewise, a 0.5% solution of Carbopol® when mixed with 0.1%, 0.2%, 0.3%, 0.5% and 1.0% 3-alanine respectively produces gels with pH values of 4.1, 4.3, 4.4, 4.5, 4.75 and viscosity values of 15,000, 20,000,22,500, 24,000 and 26,000 cps, respectively.

In another preferred embodiment of the present invention, other polymers composed of pendent carboxylic acid groups can be used when properly buffered with soluble amino acids. The carboxylic moiety may be any polymer having such groups and complying with the other parameters hereinunder described will work in this invention. Examples include poly(acrylic) and poly(methacrylic) acids and mixtures thereof and copolymers of (meth)acrylic acid with other comonomers. However, preferred polymers are of the "comb" type, that is, polymers comprising a polymer backbone (whose chemical nature is irrelevant to the working of the invention) with carboxyl group-containing moieties pendant therefrom. The moieties may be up to 100 units long and may comprise monomer residues of, for example (meth)acrylic acid, maleic acid or fumaric acid. It is preferred that the moieties be completely made up of (meth)acrylic acid residues. It will be understood that those having ordinary skill in the art having the present disclosure before them will understand what effective amounts of said polymers and soluble amino acids to mix.

In accordance with the present invention, the step of iontophoretically delivering the medicament may include the step of iontophoretically delivering the medicament using a negative polarity current between approximately 1 mA and approximately 4 mA for a period of between approximately 1 min. and approximately 20 min. It is also contemplated that these ranges can be, for example, 0.1 mA to 4 mA and from min to several hours.

The present invention is further directed to a transdermal iontophoretic device for delivering a medicament to an affected area of a living subject's body, comprising an active electrode assembly associated with a matrix, wherein the matrix includes a medicament capable of treating inflammation, immune suppression, etcetera, a viscosity enhancer to store the medicament, and a rehydrating agent to facilitate homogeneous hydration of the matrix.

### BRIEF DESCRIPTION OF THE DRAWINGS

The invention will now be described with reference to the drawings wherein:
FIG. 1 of the drawings is a cross-sectional schematic representation of a first embodiment of an iontophoretic device fabricated in accordance with the present invention; and
FIG. 2 of the drawings is a cross-sectional schematic representation of a first embodiment of an iontophoretic device fabricated in accordance with the present invention showing the association of a counter electrode assembly and an energy source.

### DETAILED DESCRIPTION OF THE INVENTION

While this invention is susceptible of embodiment in many different forms, there is shown in the drawings and will herein be described in detail several specific embodiments with the understanding that the present disclosure is to be considered as an exemplification of the principles of the invention and is not intended to limit the invention to the embodiments illustrated.

It will be understood that like or analogous elements and/or components, referred to herein, may be identified throughout the drawings with like reference characters.

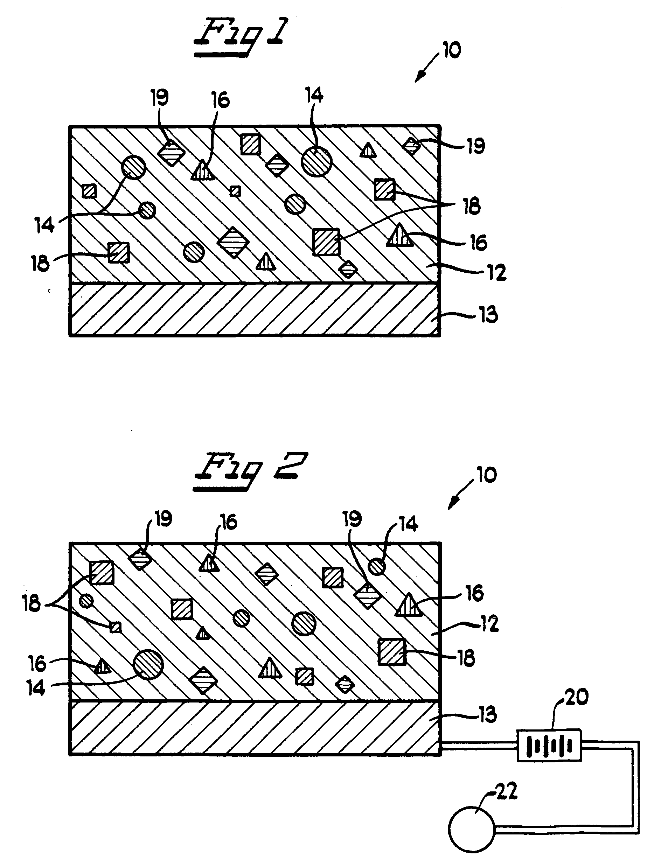

Referring now to the drawings and to FIG. 1 in particular, a first embodiment of an iontophoretic delivery device 10 is shown, which generally comprises active electrode assembly 13 and matrix 12. It will be understood that FIG. 1 is merely a cross-sectional schematic representation of an iontophoretic device. As such, some of the components have been distorted from their actual scale for pictorial clarity. Iontophoretic device 10 may be configured for application to the skin of a living subject. As will be discussed in detail below, iontophoretic device 10 is configured for delivering one or more medicament(s), which are capable of treating inflammation, immune suppression, and etcetera, and among other localized effect, around the skin of a living subject. Matrix 12 within iontophoretic device 10 is homogeneous in form having buffering agent 16 neutralized with any one of amino acids to maintain the pH above 4.0, and to deviate the pH within 4.1 to 4.9. Matrix 12 also contains other types of aqueous medications in order to treat affected areas of a living subject, which may be at least one of tissue inflammations. Further, the homogeneous matrix 12 comprises viscosity enhancer 18 to enhance the viscosity of matrix 12 to store the aqueous medicaments in place so that the medicaments are effectively administered iontophoretically. Rehydrating agent 19 is also added in the matrix so that matrix 12 is constantly rehydrated, allowing the buffering agent in matrix 12 to resist extreme changes in pH due to hydrolysis during operation of iontophoretic device 10.

Iontophoretic delivery device 10 of the present invention includes a preferred embodiment in which a polymer having pendant carboxylic acid moieties work in concert with any soluble amino acids to maintain the pH of polymeric gel matrix 12 above 4.0 and adjust the pH from 4.1 to 4.9. In particular, the pH is preferably at 4.5 for iontophoretic delivery of a medicament to treat affected areas of living subject, such as preventing skin inflammation, for one. Therefore, in a preferred embodiment, the polymeric gel matrix may include poly (methylvinylether maleic acid), an anionic polymer, coupled with any soluble amino acids which aid in neutralizing the said polymer to cause the pH in the matrix to maintain above 4.0, and adjust from approximately 4.1 to approximately 4.9, and preferably, at 4.5, for iontophoretic delivery of a medicament. Further, in this preferred embodiment, viscosity enhancers such as hydroxy ethyl cellulose and hydroxy propyl cellulose are provided in matrix 12 to store medicament 14 in the device. Rehydrating agent 19 such as Tween-A20 may be incorporated into matrix 12 to facilitate a homogeneous make up to ensure constant hydration. This rehydration within the system will ameliorate the prior art limitations which caused skin irritation and other negative harms limited the iontophoretic delivery device uses. Therefore, this embodiment allows for a suitable alternative for existing metal/metal chloride and ion exchange resin systems.

Active electrode assembly 13 generally comprises a conductive material, which upon application of an electrical potential difference thereto, drives an ionic medicament (i.e. an anionic or cationic medicament), received from the matrix and delivers the medicament into predetermined tissues and surrounding area. It will be understood that active electrode assembly 13 may comprise an anode or a cathode depending upon whether the medicament is cationic or anionic in form. The preferred embodiment uses anionic medicament. It will be further understood that active electrode assembly may include a low current density, open-faced or a smaller, high current density electrode. As would be readily understood to those with ordinary skilled in the art, any one of number of conventional active electrode assemblies is contemplated for use in accordance with the present invention. The only contemplated limitation relative to active electrode assembly is that it must be geometrically and compositionally compatible for transdermal applications of living subjects, most relevantly, humans.

Matrix 12 extends contiguously from active electrode 13, and is preferably fabricated from a material capable of temporarily retaining the medicament in solution. The solution may also contain supplemental agents, such as electrolyte, stability additives, preserving additives, pH regulating buffers, etc. Matrix 12 may comprise, for example, a natural or synthetic amorphous member, a natural or synthetic sponge pad, a natural or synthetic lint free pad, a natural or synthetic low particulate member - just to name a few. Indeed, numerous other materials that would be known to those with ordinary skill in the art having the present disclosure before them are likewise contemplated for use, including monolithic or layered viscoelastic solid hydro gels or liquid reservoirs contained with microporous membranes. As with active electrode assembly, the only contemplated limitation relative to the matrix is that it must be geometrically and compositionally compatible for transdermal applications of living beings, most relevantly, humans.

Buffering agent 16 is associated within matrix 12 to ensure consistent pH level throughout the iontophoretic delivery of medicaments 14 to affected localized areas of a living subject's body. Buffering agent 16 comprises acrylates and similar polymers having pendant carboxylic acid moieties. The carboxylic moiety may be a carboxyl group, and any polymer having such groups and complying with the other parameters hereinunder described will work in this invention. Examples include poly (acrylic) and poly (methacrylic) acids and mixtures thereof and copolymers of (meth)acrylic acid with other comonomers. However, preferred polymers are of the "comb" type, that is, polymers comprising a polymer backbone (whose chemical nature is irrelevant to the working of the invention) with carboxyl group-containing moieties pendant therefrom. The moieties may be up to 100 units long and may comprise monomer residues of, for example (meth)acrylic acid, maleic acid or fumaric acid. It is preferred that the moieties be completely made up of poly (methylvinyl ether maleic) acid, otherwise known as Gantrez®. As would be readily understood to those with ordinary skill in the art, polymers having pendant carboxylic acid moieties are well known, such as Carbopol® and Gantrez® - just to name a few. These polymers have anionic characteristics, which provide iontophoretic delivery device 10 a constant pH level throughout the administration of the medicaments via the delivery system. In this preferred embodiment, these polymers having the above characteristics will be used, and depending on the effective amount of buffering agent 16 used, the pH level will be maintained approximately within 4.1 to 4.9, and preferably, at 4.5 for operation of the iontophoretic device. The effective amount to be used will be familiar to those ordinary skilled in the art having this disclosure before them as a guide. Also to keep in mind, for purposes of this disclosure, it will be understood to those with ordinary skill in the art that buffering agent 16 comprising acrylates and similar polymers having pendant carboxylic acid moieties may comprise anodic or cathodic characteristics depending upon whether the medicament to be administered is anodic or cathodic in form.

An aqueous or mixed aqueous of medicament is retained within the matrix via introducing the viscosity enhancers 18 such as hydroxy ethyl cellulose and/or hydroxy propyl cellulose which functions to increase the viscosity of matrix 12. In order to ensure a homogeneous mixture within matrix 12, rehydrating agent 19 is provided in matrix 12. Those with ordinary skill in the art will readily optimize effective dosages without undue experimentation.

In a preferred embodiment of the present invention, matrix 12 may include poly (methyvinyl ether maleic acid), derivatives and analogs thereof.

As is shown in FIG. 2, iontophoretic device 10 may also include counter electrode 22 assembly and energy source 20. Counter electrode 22 assembly may be housed within iontophoretic device 10, or alternatively, may be remotely associated with iontophoretic device 10 via conventional electrical conduit 20. Counter electrode 22 assembly is configured for completing an electrical circuit between active electrode assembly 13 and energy source 20. As with active electrode 13, counter electrode 22 may comprise an anode or cathode depending upon whether the medicament is cationic or anionic in form. As would be readily understood to those having ordinary skill in the art, any one of a number of counter electrodes is contemplated for use in accordance with the present invention.

Similarly, counter electrode 22 assembly and energy source 20 may be housed within iontophoretic device 10, or alternatively, may be remotely associated with iontophoretic device 10 via conventional electrical conduit. The energy source for generating an electrical potential difference preferably provides for an initial higher voltage during current ramp-up to break down higher initial tissue resistance as in commercial power supply units used for transdermal iontophoresis.

In operation, the present invention can deliver a medicament in accordance with the following process. Initially, the polymeric gel matrix 12 in iontophoretic device 10 is pre-loaded with medicament 14, pH buffering agents 16 having pendant carboxylic moieties, viscosity enhancer 18, and rehydrating agent 19. Upon loading these elements into matrix 12 with iontophoretic device 10 as a housing, the device is placed on a living subject's body to initiate the delivery of the medicament via iontophoresis technique. One example includes 0.4% dexamethasone (4 mg/ml).

For purposes of the present disclosure, energy source 20 may include one or more primary or secondary electrochemical cells. While specific examples of the energy source have been disclosed, for illustrative purposes only, it will be understood that other energy sources known to those having ordinary skill in the art having the present disclosure before them are likewise contemplated for use.

The foregoing description merely explains and illustrates the invention and the invention is not limited thereto except insofar as the appended claims are so limited, as those skilled in the art who have the disclosure before them will be able to make modifications without departing the scope of the invention.

## Claims

1. An iontophoretic device for delivering a medicament to treat an affected area of a living subject's body wherein said iontophoretic device is configured for transdermal application, wherein the device comprises:
- a buffering agent associated with a polymeric gel matrix; wherein
- the buffering agent further comprises polymers having pendant carboxylic acid moieties which can maintain pH of the gel matrix from approximately 4.1 to approximately 4.9, and, preferably pH 4.5, for iontophoresis applications;
- a viscosity enhancer associated with the polymeric gel matrix to temporarily store the medicament in the matrix;
- a rehydrating agent associated with the polymeric gel matrix to facilitate homogeneous hydration in the matrix;
- a medicament associated with the polymeric gel matrix; and
- an active electrode assembly associated with the polymeric gel matrix configured for iontophoretically delivering the medicament to the affected area of the living subject's body.

2. The iontophoretic device according to claim 1, wherein the medicament associated with the polymeric gel matrix may be selected from the group consisting of:
- Dexamethasone; and
- Lidocaine.

3. The iontophoretic device according to claim 1, wherein the medicament may be selected from the group consisting of the following chemical structures:

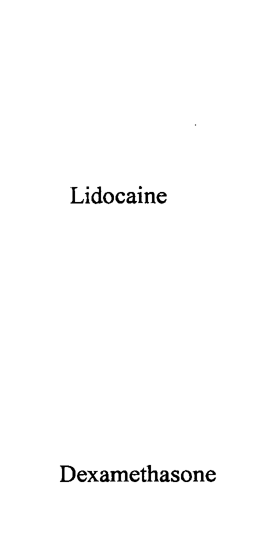

4. The iontophoretic delivery device according to claim 1, further comprising:
- a counter electrode assembly, wherein the counter electrode assembly is configured for completing an electrical circuit between the active electrode assembly and an energy source; and
- an energy source for generating an electrical potential difference.

5. The iontophoretic device according to claim 1, wherein the buffering agent is any neutralized acrylate polymer having the following structure; 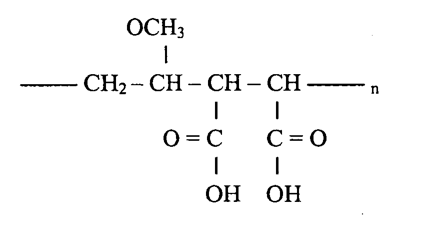
- wherein an effective amount of said polymer maintains the pH above 4.0 and adjust the pH between approximately 4.1 to approximately 4.9, and preferably pH 4.5 for effectively delivering the medicament to treat affected area of a living body's subject.

6. The buffering agent according to claim 5, wherein the neutralized acrylate polymer is neutralized with any one of soluble amino acids having the following chemical structures: 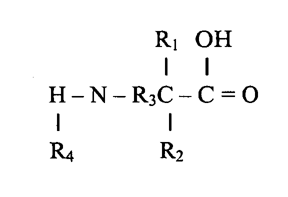
- wherein, R1, R2, and R4 are the same or different and comprise H, methyl, ethyl, propyl, butyl, aromatic and combinations thereof; and
- R3 is the same or different and comprises a direct carbon-nitrogen bond, methylene, propylene, butylene, aromatic and combinations thereof.

7. The iontophoretic device according to claim 1, wherein the viscosity enhancer capable of temporarily storing the medicament is hydroxy ethyl cellulose.

8. The iontophoretic device according to claim 1, wherein the rehydrating agent capable of facilitating hydration of the matrix is a Tween-A20.

9. The iontophoretic device according to claim 1, wherein the active electrode assembly includes an open-faced or high current density electrode.

10. A method for treating an affected area of a living subject's body, wherein the method comprises the steps of:
- associating a medicament with a matrix in an iontophoretic delivery device;
- providing an effective amount of pH buffering agents to the matrix;
- attaining high buffer capacity by providing a viscosity enhancer to the matrix;
- adding a rehydration agent to the matrix to facilitate homogeneous hydration within the matrix;
- positioning at least a portion of the iontophoretic device on the affected area of a living subject; and
- iontophoretically delivering the medicament to the affected area of the living subject to minimize skin inflammation.

11. The method according to claim 10, wherein the matrix configured for iontophoretically delivering the medicament to the affected area of the living subject's body further comprises the steps of:
- means for maintaining a pH above 4.0; and
- means for deviating a pH in the ranges from approximately 4.1 to approximately 4.9, and, preferably pH 4.5, for transdermal iontophoretic delivery of the medicament to the affected area of the limiting subject's body.

12. The method according to claim 10, wherein the step of administering a medicament may be selected from the group consisting of the following chemical structures: Dexamethasone

13. The method according to claim 10, wherein the step of administering a medicament with the living subject includes the step of providing pH buffering agents and any one of amino acids wherein both work in concert to maintain pH above pH 4.0 and assure minimal deviations from a pH of about pH 4.5.

14. The method according to claim 10, wherein the step of administering a medicament with the living subject includes the step of providing any one of viscosity enhancers to store the medicament in place of the matrix.

15. The method according to claim 10, wherein the step of administering a medicament with the living subject includes the step of providing rehydrating agents to facilitate hydration of the matrix, wherein the rehydrating agent further comprises Waterlock A220.
